# EUROPÄISCHE PATENTANMELDUNG

(11) **EP 1 045 146 A2**
(43) Veröffentlichungstag der Anmeldung: **18.10.2000**
(21) Anmeldenummer: 00108263.5
(22) Anmeldetag: 14.04.2000
(51) Int. Cl.: F04B 43/12

(54) **Medizinische Dosierpumpe**

(30) Priorität: 14.04.1999 DE 19916876
(71) Anmelder: Micheler, Clemens, 82319 Starnberg (DE)
(72) Erfinder: Micheler, Clemens, 82319 Starnberg (DE)
(74) Vertreter: Köster, Hajo

(57) **Zusammenfassung**

Bereitgestellt wird eine außen am Körper tragbare, medizinische Dosierpumpe, insbesondere Peristaltikpumpe, die aufgebaut ist aus einer Pumpe 11, die einerseits über eine Zuführungsleitung 27 mit einem Behältnis 24 für das zu verabreichende flüssige Medikament und andererseits über eine Abführungsleitung 28 mit einem Anschluß 25 für einen Katheter oder ähnlichem verbunden ist, und mit einem die Pumpe 11 antreibenden Motor sowie einer Energiequelle und Steuerungsmitteln für den Motor. Diese Dosierpumpe zeichnet sich dadurch aus, daß die Pumpe 11 zusammen mit der Zuführungsleitung 27 und Abführungsleitung 28 und dem Behältnis 24 in einer Pumpeneinheit 2 angeordnet ist, der Motor, die Energiequelle und die Steuermittel zusammen in einer Antriebseinheit 1 untergebracht sind und die Pumpeneinheit 2 mit der Antriebseinheit 1 unter Herstellung einer Wirkverbindung zwischen Motor und Pumpe 11 lösbar zusammengefügt werden kann. Bei der Pumpeneinheit handelt es sich insbesondere um eine wegwerfbare Einwegeinheit. Die teuere Antriebseinheit kann somit mit unterschiedlichen, billiger herzustellenden Pumpeneinheiten kombiniert und wiederverwendet werden.

## Beschreibung

Die Erfindung betrifft eine außen am Körper tragbare, medizinische Dosierpumpe, insbesondere Peristaltikpumpe, aufgebaut aus einer Pumpe, die einerseits über eine Zuführungsleitung mit einem Behältnis für das zu verabreichende, flüssige Medikament und andererseits über eine Abführungsleitung mit einem Anschluß für einen Katheter oder ähnlichem verbunden ist, und mit einem die Pumpe antreibenden Motor sowie einer Energiequelle und Steuerungsmitteln für den Motor.

Die Zufuhr von Medikamentenspezialitäten verlangt in zahlreichen Situationen die kontrollierte Verabreichung von teilweise hochpotenten Medikamenten- und Infusionslösungen. Daher gehören Infusions- und Infusionsspritzenpumpen inzwischen zu den medizinisch-technischen Geräten mit der größten Verbreitung bei der stationären Patientenversorgung. Die damit im Zusammenhang stehenden Vorteile und auch Probleme sind zusammenfassend beschrieben u.a. in dem Artikel "Pumpengetriebene Applikation von Medikamenten- und Infusionslösungen" von J. H. Hähnel et al. in "Medizintechnik" 111, 4/91, S. 144 bis 147.

Zu den für diese Zwecke einsetzbaren Pumpen gehört beispielsweise die sogenannte Peristaltikpumpe, bei der das zu verabreichende Volumen durch eine Kompression des Infusionsschlauches zum Patienten gefördert wird, wobei diese Förderung durch eine Rollenpumpe bzw. Schlauchpumpe bewirkt wird.

Derartige Peristaltikpumpen mit einem deformierbaren Schlauch bzw. Rollenpumpen sind auch Gegenstand verschiedener Patentanmeldungen; beispielsweise sei hier verwiesen auf die FR 2 383 333 A1 und FR 2 417 025 A1. Eine Peristaltikpumpe mit einem abnehmbaren Pumpengehäuse ist beispielsweise beschrieben in der FR 2 599 434 A1.

Eine weitere Rollenpumpe, bei der die Pumpe aus zwei zusammenfügbaren und wieder auseinandernehmbaren Einheiten besteht, ist in der EP 0 388 269 A1 beschrieben. Durch die dort vorgeschlagene Lösung soll erreicht werden, daß der deformierbare Schlauch entlastet und somit wieder frei durchgängig wird, damit eine Sterilisation dieses Schlauches erfolgen kann. Diese Problemstellung spielt eher im stationären Bereich eine Rolle, wenn es erforderlich ist, eine Pumpe der hier in Rede stehenden Art für einen neuen Patienten zum Einsatz zu bringen.

Es besteht nun ein dringendes Bedürfnis danach, die stationäre Versorgung von Patienten zugunsten einer ambulanten Versorgung zurückzudrängen, um somit u.a. Kosten im Gesundheitswesen zu sparen. Allerdings steht diesem Wunsche das Erfordernis gegenüber, daß viele Patienten ständig oder intermittierend bzw. pulsatil mit Medikamenten versorgt werden müssen. Als Beispiele hierfür lassen sich nennen: Insulin, Morphin, L-Dopa, Heparin, Zytostatika usw. Für diesen Zweck sind am Körper tragbare Peristaltikpumpen entwickelt worden, die quasi miniaturisierte Pumpen darstellen. Als eigentliche Pumpen werden dabei insbesondere Rollenpumpen zur Anwendung gebracht, da diese dem Miniaturisierungszweck gut zugänglich sind.

Die bisher bekannten, am Körper tragbaren Pumpen der hier in Rede stehenden Art sind jedoch entweder sehr aufwendig und teuer oder können nicht wiederverwendet werden oder zum Einsatz unterschiedlicher Medikamente eingesetzt werden.

Aufgabe der vorliegenden Erfindung ist es daher, eine einfachere und verbesserte, am Körper tragbare, medizinische Dosierpumpe, insbesondere Peristaltikpumpe, bereitzustellen.

Gelöst wird diese Aufgabe durch die Lehre der Ansprüche.

Eine der wesentlichen Aspekte der vorliegenden Erfindung liegt somit darin, daß die erfindungsgemäße Pumpe aus zwei diskreten Einheiten besteht, nämlich einer Pumpeneinheit und einer Antriebseinheit. Diese beiden Einheiten können je nach Bedarf miteinander verbunden bzw. zusammengefügt werden, wobei eine Wirkverbindung zwischen Motor und Pumpe hergestellt wird. Mit dem Ausdruck "Wirkverbindung" soll zum Ausdruck gebracht werden, daß der Motor im zusammengebauten Zustand der beiden Einheiten in der Lage ist, die Pumpe anzutreiben. Zu diesem Zwecke kann man beispielsweise die aus dem Motor herausführende Welle als Vierkant ausgestalten, der in eine entsprechende Öffnung oder Bohrung in der Pumpe unter Herstellung eines Formschlusses eingreift, so daß beim Drehen der Motorwelle auch die Pumpe angetrieben wird.

Die Verbindung zwischen der Pumpeneinheit und der Antriebseinheit muß dabei dergestalt sein, daß sich diese beiden Einheiten wunschgemäß wieder voneinander lösen lassen und natürlich auch wieder miteinander verbinden lassen. Die Mittel zum Verbinden der beiden Einheiten können dabei vielgestaltig sein. So kann eine Einheit mit Zapfen ausgestattet sein, die in entsprechende Ausnehmungen in der anderen Einheit eingreifen. Diese Verbindungen können clipartig oder auch bügelartig ausgestaltet sein. Derartige Verbindungsmöglichkeiten sind dem Fachmann gut bekannt und bedürfen daher keiner weiteren Erläuterungen.

Die Antriebseinheit weist dabei den insbesondere elektrisch angetriebenen Motor, eine Energiequelle für diesen Motor, beispielsweise Batterien oder Akkus, und die Steuerungsmittel für den Motor auf. Durch die Steuerungsmittel wird festgelegt, um wieviele Umdrehungen in welcher Zeit sich der Motor dreht. Dadurch wird natürlich auch die Dosierung des Medikamentes festgelegt. So entspricht beispielsweise bei einer Rollenpumpe eine Umdrehung der Motorwelle eine bestimmte Dosierung, die natürlich auch abhängt von den Dimensionen der Rollenpumpe und insbesondere von dem Schlauchdurchmesser. Die Steuermittel können beispielsweise derart eingestellt werden, daß die Motorwelle intermedierend eine bestimmte Umdrehungsanzahl durchführt, beispielsweise alle halbe Stunde, alle Stunde usw. Die Steuermittel können auch eine Uhr oder eine andere Zeitmeßeinrichtung aufweisen, so daß beispielsweise die Medikamentenabgabe tagsüber stärker oder geringer ist als nachts usw. Zweckmäßigerweise weisen daher die Steuermittel einen Chip auf, der insbesondere programmierbar ist, um den zeitlichen Ablauf der Drehung der Welle des Motors, bei dem es sich beispielsweise um einen Schrittmotor handeln kann, in seiner zeitlichen Abfolge festzulegen.

Die Steuermittel weisen vorzugsweise einen sogenannten Bolus-Knopf auf, bei dessen Betätigung durch den Patienten eine zusätzliche Arzneimenge bei Bedarf verabreicht wird. Dies gilt beispielsweise für einen Krebspatienten, der zur Schmerzbehandlung Morphin verabreicht bekommt. Im Falle eines Schmerzschubes kann dann eine zusätzliche Morphinmenge, die vorher eingestellt werden kann, verabreicht werden. Gleiches gilt für einen Diabetiker, bei dem eine zusätzliche Insulinmenge verabreicht werden soll.

Die eigentliche Pumpe ist zusammen mit der Zuführungsleitung von dem Behältnis für das zu verabreichende Medikament und der Abführungsleitung zu einem Anschluß für einen Katheter oder ähnlichem in einer diskreten, separaten Pumpeneinheit angeordnet.

Bei dem Anschluß für einen Katheter kann es sich beispielsweise um einen Luer-Lock-Anschluß handeln. Dieser Anschluß wird dann mit dem Katheter verbunden, der insbesondere für eine subkutane Verabreichung des flüssigen Arzneimittels dienen kann.

Bei dem Behältnis kann es sich im einfachsten Fall um eine Ausnehmung oder Bohrung in einem Behälter handeln, in das das zu verabreichende Medikament eingefüllt und verschlossen wird, beispielsweise mit einem Gummistopfen oder ähnlichem. Die Innenmantelfläche des Behältnisses kann dabei silikonisiert sein. Es ist auch möglich, eine mit dem Medikament gefüllte Kartusche in die Pumpeneinheit unter Herstellung einer Verbindung zwischen der Kartusche und der Zuführungsleitung einzuführen.

Die Art der Pumpe ist dabei nicht entscheidend. Alle bisher bekannten Pumpen für hier in Rede stehende, am Körper tragbare, medizinische Dosierpumpen, können Anwendung finden. Vorzugsweise handelt es sich dabei um eine Rollenpumpe. Dabei wird der Schlauch dieser Rollenpumpe durch Rollen oder ähnliches unter Förderung des Arzneimittels in bestimmten Abständen zusammengedrückt.

Die Pumpeneinheit weist vorzugsweise auch Mittel auf, die mit korrespondierenden Mitteln in der Antriebseinheit verbunden oder in Kontakt gebracht werden können, so daß der Antriebseinheit und somit den dort vorhandenen Steuermitteln mitgeteilt wird, welches Volumen pro Drehung der Motorwelle von der Pumpeneinheit abgegeben wird. Dieses Volumen hängt natürlich von der Dimensionierung der Rollenpumpe und insbesondere dem Durchmesser des Schlauches dieser Rollenpumpe ab. Diese Informationen können dann von den Steuermitteln der Antriebseinheit verwertet werden bzw. unter Berücksichtigung dieser Werte kann die Motordrehung für die gewünschte Volumenabgabe bestimmt werden. Natürlich muß in einem solchen Fall die Antriebseinheit auch über Mittel, beispielsweise elektrische Mittel verfügen, um diese Informationen erhalten und verarbeiten zu können.

Mit der erfindungsgemäßen Dosierpumpe ist es somit möglich, die an sich teuere Antriebseinheit mit unterschiedlichsten Pumpeneinheiten je nach zu verabreichendem Arzneimittel zu verbinden. Von den Krankenkassen und anderen medizinischen Einrichtungen muß nur eine Antriebseinheit bereitgehalten werden, die je nach Erfordernis mit der entsprechenden Pumpeneinheit verbunden wird. Bei der Pumpeneinheit handelt es sich dabei vorzugsweise um eine wegwerfbare Einwegeinheit, so daß keine Sterilisationsprobleme zu befürchten sind, wie sie bei dem eingangs abgehandelten Stand der Technik zu besorgen waren. Die Pumpeneinheit läßt sich aus kostengünstigen Kunststoffmaterialien und Plastikschläuchen usw. herstellen. Die aufwendiger herzustellende und somit kostenträchtigeren Steuermittel und der Motor sind in der wiederverwendbaren Antriebseinheit untergebracht.

Die beiden Einheiten sind vorzugsweise derart ausgestaltet, daß die Motorwelle der Antriebseinheit in die Pumpe der Pumpeneinheit unter Herstellung der Wirkverbindung einsteckbar ist. Auf diese Weise wird beim bestimmungsgemäßen Zusammenbau von Antriebseinheit und Pumpeneinheit auch die Wirkverbindung zwischen Motor und Pumpe hergestellt. Dazu kann man beispielsweise die Motorwelle aus dem Motor und aus der Pumpeneinheit herausragend ausgestalten. Bei der Welle kann es sich - wie oben geschildert - beispielsweise um eine Vierkantwelle oder um eine eine Zahnung aufweisende Welle handeln. Die Pumpeneinheit weist dann eine dazu korrespondierende Aufnahme auf, in die diese Welle unter Herstellung eines Formschlusses und somit einer Wirkverbindung eingesteckt bzw. eingeführt werden kann.

Die Erfindung wird im folgenden anhand der Zeichnungen näher erläutert, welche eine bevorzugte Ausführungsform skizzenhaft und nicht maßstabsgetreu wiedergeben. Von den Zeichnungen zeigen:

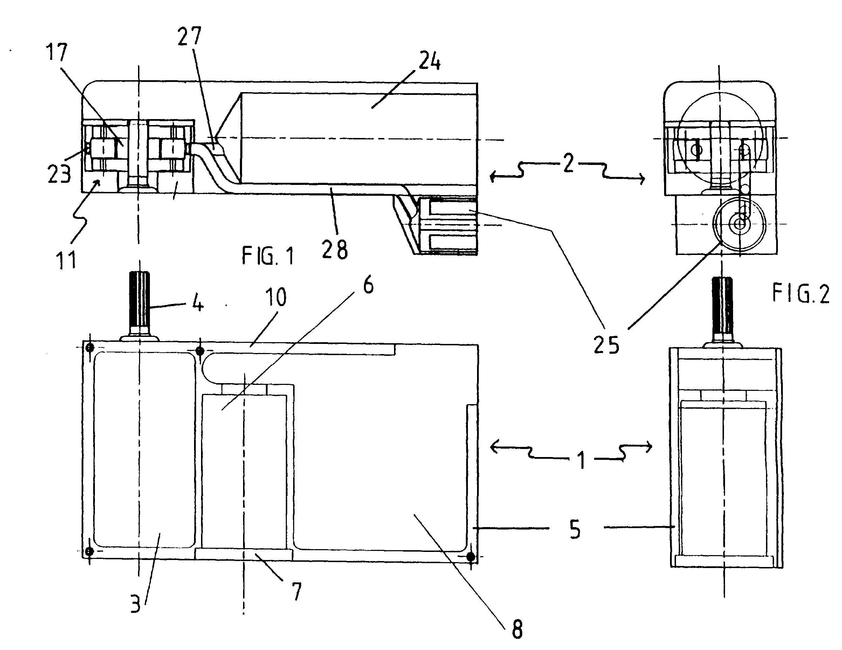

- Fig. 1: eine schematische Längsschnittansicht einer aus einer Antriebseinheit und einer Pumpeneinheit bestehenden erfindungsgemäßen Dosierpumpe im auseinandergebauten Zustand;
- Fig. 2: eine Seitenansicht/Querschnittsansicht der in der Fig. 1 gezeigten Pumpe, von rechts in der Fig. 1 betrachtet,
- Fig. 3: eine der Fig. 1 analoge Ansicht, wobei die Dosierpumpe jedoch zusammengebaut ist und
- Fig. 4: eine Explosionsansicht der erfindungsgemäßen Dosierpumpe.

Die erfindungsgemäße Dosierpumpe besitzt eine Antriebseinheit 1 und eine Pumpeneinheit 2, die zu der erfindungsgemäßen Dosierpumpe zusammengefügt werden können.

Die Antriebseinheit 1 weist ein Gehäuse 5 mit verschiedenen Fächern auf. Dazu gehört beispielsweise das Motorfach 3, in dem ein nicht gezeigter Motor untergebracht ist. Die Welle 4 dieses Motors ist aus dem in etwa quaderförmigen Gehäuse 5 hinausgeführt und stellt im Außenbereich eine Zahnwelle bzw. ein Ritzel dar.

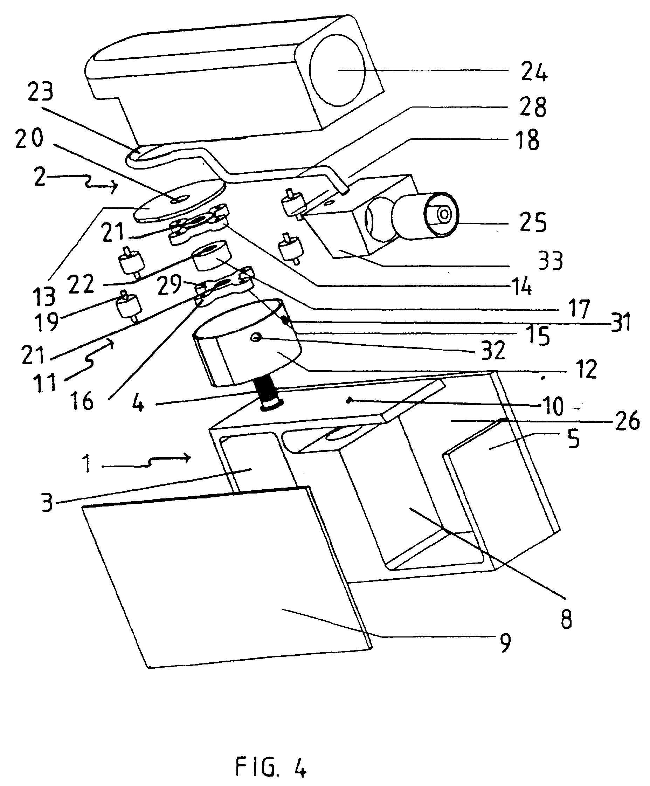

Im Gehäuse 5 befinden sich ferner ein Batteriefach 6 zur Aufnahme von üblichen Batterien oder Akkus, die auf geeignete Weise mit dem Motor verbunden sind. Um die Batterien bzw. Akkus wechseln zu können, ist das Gehäuse 5 an seinem Boden mit einem Deckel 7 ausgestattet, der herausnehmbar ist und zum Verschließen des Batteriefaches 6 dient. In dem Elektronikfach 8 des Gehäuses 5 können die in ihren Einzelheiten nicht gezeigten Steuermittel sowie der gegebenenfalls programmierbare Chip usw. angebracht sein. Das Gehäuse 5 ist somit in verschiedene Fächer bzw. Abteile unterteilt und zu einer Seite hin offen, die durch den Seitendeckel 9 (man vergleiche Fig. 4) verschlossen werden kann.

Die Pumpeneinheit 2 stellt in etwa einen Stab mit einem in etwa quadratischen Querschnitt dar, der auf die obere Seitenwand 10 des Gehäuses 5 aufgesetzt werden kann. In der Pumpeneinheit 2 ist eine Rollenpumpe untergebracht, die insgesamt mit 11 bezeichnet ist. Wie insbesondere aus der Fig. 4 ersichtlich ist, besitzt diese Rollenpumpe ein in etwa zylindrisches Pumpengehäuse 12, das oben durch den Deckel 13 abgeschlossen wird. In diesem Pumpengehäuse 12 befinden sich ein oberer Stern 14 und ein unterer Stern 15 mit jeweils vier sich in etwa radial erstreckenden, gleichmäßig angeordneten Armen 16. Zwischen diesen Sternen 14, 15 ist ein Zwischenring 17 angeordnet. Sowohl die beiden Sterne als auch der Zwischenring weisen jeweils eine zentrische, durchgehende Bohrung 21 bzw. 22 auf, in die hinein sich die Welle 4 erstreckt, wenn die Pumpeneinheit 12 mit der Antriebseinheit 1 verbunden wird. Die Mittelachse dieser Bohrungen 21, 22 ist somit im zusammengebauten Zustand der Dosierpumpe axial fluchtend mit der Achse der Welle 4.

Bei der in den Fig. dargestellten Ausführungsform besitzen die Seitenmantelfläche der Bohrungen 21, 22 der Sterne 14, 15 und des Zwischenringes 17 in Axialrichtung verlaufende Nuten bzw. Rillen. Mit anderen Worten, die Welle 4 kämmt mit dem oberen Stern 14, dem unteren Stern 15 und dem Zwischenring 17, so daß ein Formschluß und dadurch eine Wirkverbindung zwischen dem Motor und der Pumpe 11 hergestellt wird. Beim Drehen der Welle 4 drehen sich auch die Sterne 14, 15 sowie der Zwischenring 17.

Zwischen den Armen 16 des oberen und des unteren Sternes 14, 15 sind zylindrische Rollen 18 angeordnet, die auf ihren beiden kreisförmigen Stirnflächen jeweils einen zentrisch angeordneten Zapfen 19 besitzen, der in dazu korrespondierenden Bohrungen bzw. Aufnahmen 29 in den Armen 16 des oberen und des unteren Sterns 14, 15 gelagert sind.

In das Pumpengehäuse 12 ist ein zusammendrückbarer Schlauch 23 eingesetzt. Der zuführende Schlauchabschnitt bzw. die Zuführungsleitung 27 wird durch die Öffnung 31 im Pumpengehäuse 12 hineingeführt, während der ableitende Schlauchabschnitt bzw. die Abführungsleitung 28 durch die Öffnung 32 im Pumpengehäuse 12 hinausgeführt wird, man vergleiche insbesondere Fig. 4.

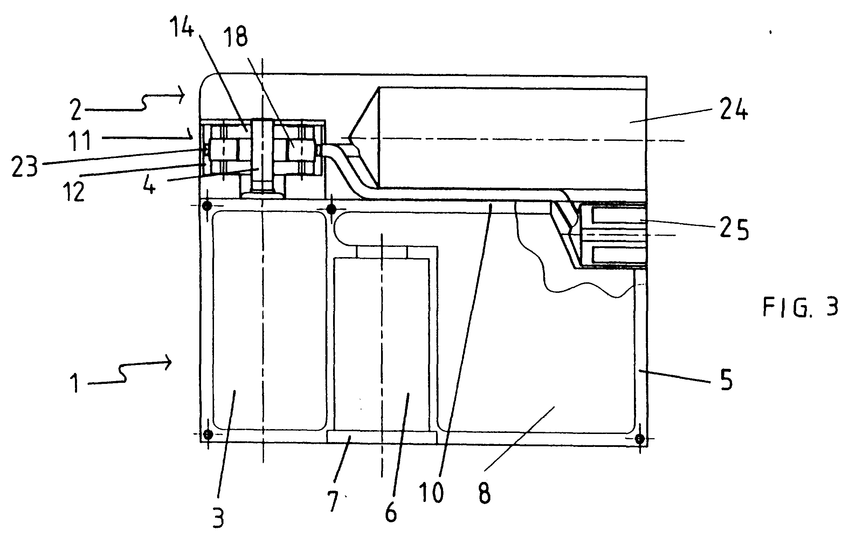

Der Schlauch 23 ist zuführungsseitig mit einem Behältnis 24 (man vergleiche Fig. 3) verbunden, in den das zu verabreichende Medikament eingefüllt wird und dann mit beispielsweise einem Gummistöpsel (nicht gezeigt) verschlossen wird. Abführungsseitig ist der Schlauch 23 mit einem Luer-Lock 25 zum Anschluß eines Katheters oder ähnlichem verbunden.

Beim Drehen der Motorwelle 4 und somit der Sterne 14, 15 wird der Schlauch durch die Rollen 18 gegen die Innenwandung des Pumpengehäuses 12 gedrückt und dabei komprimiert, so daß die im Schlauch 23 befindliche Flüssigkeit bzw. das zu verabreichende Arzneimittel durch Kompression dieses Schlauches 23 in Richtung des Luer-Locks 25 gefördert wird.

Die Ausgestaltung der Rollenpumpe ist im übrigen üblicher und bekannter Art. Es können auch andere bekannte Pumpen zum Einsatz gebracht werden, die denselben Zweck erfüllen.

Sowohl die Antriebseinheit 1 bzw. dessen Gehäuse 5 zusammen mit dem unteren Deckel 7 und dem Seitendeckel 9 als auch die Pumpeneinheit 2 mit den verschiedenen funktionalen Teilen der Pumpe 11 sind vorzugsweise aus einem Kunststoff gefertigt, so daß das Gewicht der fertigen Einheiten möglichst gering ist und vom Patienten kaum als Belastung empfunden wird. Bei dem Schlauch 23 kann es sich beispielsweise um einen Silikonschlauch oder auch um einen Gummischlauch oder ähnliches handeln.

Die Antriebseinheit 1 und die Pumpeneinheit 2 können ohne weiteres durch Aufeinandersetzen zusammengefügt werden, da die Längsachse der Welle 4 mit den Längsmittelachsen der zentrischen Bohrungen 21,22 in den Sternen 14, 15 und dem Zwischenring 17 fluchten. Zweckmäßigerweise weist auch der Deckel 13 eine zentrische Bohrung oder Aufnahme 20 auf, in das das freie Ende der Welle 4 im zusammengebauten Zustand zu liegen kommt und damit eine Lagerung erfährt.

Zum Zusammenfügen bzw. Zusammenbauen von Pumpeneinheit 2 und Antriebseinheit 1 ist es lediglich erforderlich, die Pumpeneinheit 2 auf die Welle 4 aufzuschieben, bis die Pumpeneinheit 2 auf der oberen Seitenwand 10 des Gehäuses 5 der Antriebseinheit zu liegen kommt. Dabei verschließt das Luer-Lock 25 mit der Einheit 33, in der es untergebracht ist, die Öffnung 26 des Gehäuses 5 der Antriebseinheit 1 und es wird insgesamt eine Pumpe mit in etwa Quaderform erhalten, die ohne weiteres von einem Patienten außen am Körper, beispielsweise am Gürtel, getragen werden kann. Das Luer-Lock 25 muß dann mit einem Katheter oder ähnlichem verbunden werden, so daß das flüssige Arzneimittel an den Patienten verabreicht werden kann, beispielsweise subkutan oder i.v.

## Patentansprüche

1. Außen am Körper tragbare, medizinische Dosierpumpe, insbesondere Peristaltikpumpe, aufgebaut aus einer Pumpe (11), die einerseits über eine Zuführungsleitung (27) mit einem Behältnis (24) für das zu verabreichende flüssige Medikament und andererseits über eine Abführungsleitung (28) mit einem Anschluß (25) für einen Katheter oder ähnlichem verbunden ist, und mit einem die Pumpe (11) antreibenden Motor sowie einer Energiequelle und Steuerungsmitteln für den Motor,
dadurch **gekennzeichnet**,
daß die Pumpe (11) zusammen mit der Zuführungsleitung (27) und Abführungsleitung (28) und dem Behältnis (24) in einer Pumpeneinheit (2) angeordnet ist,
der Motor, die Energiequelle und die Steuermittel zusammen in einer Antriebseinheit (1) untergebracht sind und
die Pumpeneinheit (2) mit der Antriebseinheit (1) unter Herstellung einer Wirkverbindung zwischen Motor und Pumpe (11) lösbar zusammengefügt werden kann.

2. Dosierpumpe nach Anspruch 1,
dadurch **gekennzeichnet**,
daß die Pumpeneinheit (2) eine wegwerfbare Einwegeinheit darstellt.

3. Dosierpumpe nach Anspruch 1 oder 2,
dadurch **gekennzeichnet**,
daß die Pumpe (11) eine Rollenpumpe ist.

4. Dosierpumpe nach Anspruch 3,
dadurch **gekennzeichnet**,
daß der Schlauch (23) der Rollenpumpe mit der Zuführungsleitung (27) und der Abführungsleitung (28) einstückig ausgebildet ist und somit einen sich vom Behältnis (24) bis zum Anschluß (25) erstreckenden Schlauch (23) darstellt.

5. Dosierpumpe nach einem der vorhergehenden Ansprüche,
dadurch **gekennzeichnet**,
daß der Motor mit Hilfe der Steuermittel derart eingestellt und gesteuert werden kann, daß er die Pumpe (11) intermittierend antreibt.

6. Dosierpumpe nach einem der vorhergehenden Ansprüche,
dadurch **gekennzeichnet**,
daß die Steuermittel einen Bolusknopf aufweisen.

7. Dosierpumpe nach einem der vorhergehenden Ansprüche,
dadurch **gekennzeichnet**,
daß die Steuermittel einen Chip aufweisen, der das Antriebsverhalten des Motors steuert.

8. Dosierpumpe nach Anspruch 7,
dadurch **gekennzeichnet**,
daß der Chip programmierbar ist.

9. Dosierpumpe nach Anspruch 7 oder 8,
dadurch **gekennzeichnet**,
daß der Chip austauschbar ist.

10. Dosierpumpe nach einem der vorhergehenden Ansprüche,
dadurch **gekennzeichnet**,
daß die Welle (4) des Motors der Antriebseinheit (1) in die Pumpe (11) der Pumpeneinheit (2) unter Herstellung der Wirkverbindung einschiebbar oder einsteckbar ist und auch wieder entfernbar ist.
